# EUROPEAN PATENT SPECIFICATION

(11) **EP 3 266 863 B1**
(45) Date of publication and mention of the grant of the patent: **05.02.2020**
(21) Application number: 17178939.9
(22) Date of filing: 30.06.2017
(51) Int. Cl.: C12N 1/06, C12N 1/16, A23K 10/16, A23L 33/14

(54) **COMPOSITION SUBSTANTIALLY CONSISTING OF YEAST CELL WALLS AND PROCESS FOR THE PREPARATION THEREOF**
ZUSAMMENSETZUNG, DIE IM WESENTLICHEN AUS HEFEZELLWÄNDEN BESTEHT, UND VERFAHREN ZUR HERSTELLUNG DAVON
COMPOSITION SENSIBLEMENT CONSTITUÉ DE PAROIS DE CELLULES DE LEVURE ET SON PROCÉDÉ DE PRÉPARATION

(30) Priority: 04.07.2016 IT 201600069379
(43) Date of publication of application: 10.01.2018
(73) Proprietor: PROSOL S.p.A., 24040 Madone (BG) (IT)
(72) Inventor: BONVICINI, Daniele, 20052 Monza (MB) (IT); LISSONI, Stefano, 24030 Mapello (BG) (IT); TOGNI, Ermanno, 24030 Presezzo (BG) (IT)
(74) Representative: Ferreccio, Rinaldo

(56) References cited:
- EP-A1- 2 062 588
- WO-A1-92/07064
- WO-A1-2006/121803
- WO-A1-2009/089593
- WO-A1-2014/114342
- US-A1- 2006 257 422

## Description

### Field of application

The present invention relates to the field of food industry, as well as animal feed and zootechnics industry, in particular in areas where the consumption or the use of a yeast wall-based composition is envisaged.

More particularly, the present invention relates to a process for the production of a composition consisting of yeast cell walls, aimed at obtaining products of food, zootechnical and feed interest.

### Prior Art

It is well known that the microbial cell wall is a specialized coating structure that surrounds cell cytoplasmic membrane and has several biological functions.

It is also known that yeast cell wall ensures the cell morphology and integrity due to its particular rigidity, while remaining highly adaptable to changes of the surrounding environment.

Generally, yeast cell walls approximately represent 10-30% of cell dry weight and mainly consist of: proteins (5%) and polysaccharides (95%).

It is also known that a portion of the polysaccharides is represented by mannose residues that are covalently bound to the wall proteins (forming mannoproteins), while the remaining portion mostly consists of glucan chains (including β-glucans) which are cross-linked to each other to generate the main wall structure; the cell wall also contains chitin in an amount equal to only 1% of the polysaccharides in the yeast cells but this amount may increase varying growth conditions and based on the fungal species (Backhaus et al., A systematic study of the cell wall composition of Kluyveromyces lactis, Yeast 2010; 27: 647-660).

The use of yeast cell walls is known in zootechnical and animal feed fields, particularly for poultry, swine and bovines.

For several years, in fact, natural technologies are being sought to improve animal health and promote proper development, minimizing as much as possible the use of antibiotics, and several natural remedies promoting animal health and growth without adversely affecting the final product quality (meat, milk, butter, etc.) have been found.

It is well known that one of these natural alternatives is represented by live yeast cultures directly provided within forage or cell walls obtained from these yeasts or still a combination of these products (Broadway P., Carroll J., Burdick Sanchez N., Live Yeast and Yeast Cell Wall Supplements Enhance Immune Function and Performance in Food-Producing Livestock: A Review, Microorganisms 2015, 3, 417-427).

The fungal microorganism used for this purpose is *Saccharomyces cerevisiae,* a yeast which is widely studied and well-known to be employed in a variety of processes.

It is also known that, in such particular areas of application, the functions of this fungal organism are several: controlling the microbial population composition within animal gastrointestinal tract, preventing pathogen colonization, inhibiting toxins and mycotoxins action and modulating the immune system. (Dawson, The Application of yeast and yeast derivatives in the poultry industry, Proc. Austr. Poult. Sci. Symp. 13:100-105).

In particular, the addition of Saccharomyces cerevisiae live cell cultures into forage for ruminant animals, due to stimulation of digestive microflora, positively influences feed intake, stabilizes the rumen stomach pH and enhances the activity of the ruminant bacteria responsible for cellulose and fibers digestion, increasing ruminal fermentation efficiency (Bailey, The Benefits of Yeast Culture and Yeast Cell Wall Components in Beef Cattle, Marsyt, An Agricultural Company,2016).

The widely known on the market "SafMannan" product is a composition based on yeast cell walls with a balanced content of mannan and β-glucan, obtained from S. cerevisiae yeast and suitable for the zootechnical use for breeding cattle, swine and poultry (http: //www.princeagri.com/Phibro/Products/Catalog/Safmannan-Specialty-Product-For-Poultry-Swine-Cattle-And-Other-Species.html). The presence of β-glucans in cell walls makes these products interesting also for use in human nutrition.

β-glucans, in fact, have beneficial effects on cholesterol, inhibiting the bile acid reabsorption in ileum and jejunum, resulting in the use of endogenous cholesterol for the neosynthesis of bile salts.

In addition, the involvement of these polysaccharides in a variety of biological activities is known. For example, they improve glycemic response and modulate post-digestive secretion of gastrointestinal peptides, increasing the secretion of cholecystokinin and peptide YY and decreasing ghrelin concentration, thereby ensuring a lowering of glycemic peak and increasing of feelings of satiety, see for example US 2006/0257422.

The presence of β-glucans in the yeast cell walls also ensures a prebiotic activity of the final product because they promote selection and growth of intestinal bifidobacteria and lactobacilli bacterial flora, whose fermentation produces short chain fatty acids.

To date, yeast cell walls are obtained as by-products from yeast extract production process, starting from living yeast *Saccharomyces cerevisiae* cell cultures.

The cell walls thus obtained are characterized by a variable composition depending on the kind of process for the production of yeast extract and the different *Saccharomyces cerevisiae* yeast strains being used.

The problem underlying the present invention was to provide a composition based on yeast cell walls which is obtained by a specially suited process starting from exhausted yeast, wherein "exhausted yeast" means the yeast cells biomass recovered at the end of industrial fermentation and extraction processes (e.g. processes for the production of yeast extracts and/or ribonucleotides), see for example WO 2009/089593.

### Summary of the Invention

The problem has been solved by providing a process for the production of a composition substantially consisting of yeast cell walls, which comprises steps of:
a) dispersing exhausted yeast in an aqueous medium to obtain a first suspension of yeast cells, said exhausted yeast consisting of yeast cells biomass recovered at the end of processes for the production of yeast extracts and/or ribonucleotides;
b) treating said first suspension having a dry weight of between 5% and 12% with a proteolytic enzyme at a temperature comprised between 50°C and 70°C, for 6 to 10 hours, in an aqueous medium containing a basic compound and having a pH between 7.5 and 9.0;
c) centrifuging said first suspension obtaining a first pellet and a supernatant;
d) dispersing said first pellet in an aqueous medium, obtaining a second suspension of yeast cells;
e) centrifuging said second suspension to obtain a second pellet having a protein content comprised between 20% and 30 % on dry substance, which constitutes said composition consisting of yeast cell walls.

In one embodiment, the process also comprises a further step f) of drying the above-mentioned second pellet, which constitutes the above-mentioned composition substantially consisting of yeast cell walls obtained in step e).

In one aspect of the present invention, the above-mentioned exhausted yeast is a Crabtree negative yeast, preferably belonging to the *Kluyveromyces* genus.

Unless otherwise stated, the percentages indicated in this disclosure are intended as weight/weight percentages.

In a further aspect of the present invention, the above-mentioned step b) of treating with a proteolytic enzyme is performed at a temperature comprised between 50°C and 70°C, preferably between 55°C and 65°C, more preferably at about 60°C.

Preferably, the time period of step b) is generally at least 6 hours, preferably about 6-10 hours.

Preferably, in step b) of treating with a proteolytic enzyme, said suspension of exhausted yeast cells has a dry weight of between 5% and 12%, preferably between 7% and 12%.

Preferably, the step b) of treating with a proteolytic enzyme is performed in an aqueous medium containing a basic compound and having a pH between 7.5 and 9.0, preferably between 8.0 and 8.2.

Preferably, the basic compound is a strong base, preferably an alkaline or alkaline-earth hydroxide, and is advantageously selected from calcium hydroxide, sodium hydroxide and potassium hydroxide. Conveniently, the basic compound is sodium hydroxide.

The strong base is preferably used in the form of an aqueous solution with a concentration comprised between 20% and 40%, preferably between 25% and 35%, more preferably at a concentration of 30%.

In a further aspect, the present invention relates to a composition consisting of yeast cell walls having a content of glucans between 20% and 24%, a content of mannans between 5.5 and 7.5% and a protein content between 20% and 30% by weight, obtainable by the process described above.

In another aspect thereof, the present invention discloses a use of the above-mentioned composition as a food supplement.

In a further aspect thereof, the present invention also discloses a use of the above-mentioned composition as a feed additive.

The composition consisting of yeast cell walls, unlike the prior art compositions, involves the use of exhausted yeast masses, in particular of *Kluyveromyces* yeast, in order to advantageously use this by-product of fermentation/extraction process.

Furthermore, the composition consisting of yeast cell walls according to the present invention is advantageously characterized by a reduced protein content (protein content of less than 30%) and a high polysaccharides (particularly mannans and β-glucans) content, making this composition particularly suitable for use in animal feed and zootechnical field and possibly for human consumption.

### Detailed description of a preferred embodiment

Further features and advantages of the present invention will become apparent from the following description of an embodiment, given for purposes of illustration and not of limitation.

A by-product, commonly referred to as " exhausted yeast", is obtained from different processes for the extraction of nucleic acids from yeasts, e.g. from *Streptomyces, Kluyveromyces* and *Candida* genus yeasts. This by-product is a dense suspension, with a dry content between 17% and 22%, and with a protein content between 45% and 55% (calculated on dry substance).

The removal of proteins contained therein is possible by using a proteolytic enzyme, in particular a microbial protease applied to the exhausted yeast.

The enzymatic treatment's efficacy is affected by three main factors: temperature, pH, and exhausted yeast dilution.

The hydrolysis temperatures should be maintained within the range of 55°C to 65°C; the pH is controlled and maintained between 7 and 9; exhausted yeast dilution is advantageously performed up to a dry weight value between 7 and 12%. In fact, dry weight values greater than 12% proportionally reduce the effectiveness of enzymatic action.

The optimum enzyme dosage ranges between 1 and 3 ml (180 units of casein protease/ml of enzyme) per kg of solution obtained by diluting exhausted yeast to 7-12%.

The enzymatic reaction is carried out for a period ranging from 5 to 8 hours, controlling the pH every hour and keeping it between 8.0 and 8.1 by NaOH addition.

Subsequently, the hydrolyzed suspension of exhausted yeast is centrifuged by means of a separator centrifuge, thus obtaining a first yeast pellet of 12-16% dry substance and a waste supernatant.

The pellet thus obtained is dissolved in water to remove proteins remaining after enzymatic digestion and to bring back the suspension dry weight within the value range from 7 to 12%.

The suspension is submitted to a further centrifugation step, obtaining a waste supernatant and a second pellet having a dry weight ranging from 12 to 16%, which represents the composition of the present invention, substantially consisting of cell walls of exhausted yeast.

The protein content of the composition consisting of cell walls thus obtained ranges between 20 and 30% on the dry substance, conferring to the product a higher commercial value than the exhausted yeast as it is. The composition also has a glucans content ranging from 20% to 24% and a mannan content ranging from 5.5% to 7.5%.

The composition according to the invention is usually marketed in a dry form and said pellet is subjected to a spray drying for this purpose.

The spray drying is carried out by dispersing the composition in water to obtain a suspension with a dry weight of about 8%, which is then heated to 79-81°C, maintaining that temperature for a sufficient time to achieve spray drying.

Finally, to characterize the final product, a glucan and mannan content analysis is performed.

### EXAMPLE

In a laboratory beaker, 500 g of exhausted yeast belonging to *Kluyveromyces* genus, obtained from RNA extraction process and having the following characteristics: 17.87% dry substance, 51.39% protein on dry substance and 3.20% ash on dry substance, are weighted.

500 g of water are then added, reaching a total weight of 1000 g, and the the suspension shows a dry weight equal to 8.93%.

The beaker is then placed onto a heating-stirring plate and the suspension is kept under uniform stirring and heated up to 60°C.

The pH value of the suspension is then adjusted from 5.94 to 8.05, by adding 2.1 ml of 30% NaOH aqueous solution.

The Promod 950L (Biocatalyst) enzyme is then added in a concentration of 1.5 ml/kg.

The enzymatic reaction occurs at 60°C for 6.5 hours under stirring. During this step, at regular one-hour intervals, the pH is controlled and adjusted within a range from 8 to 8.1 by addition of a 30% NaOH solution.

The values found are shown in Table 1 below

**Table 1. pH values of the suspension during the enzymatic reaction.**

| **Hydrolysis hours** | **starting pH** | **pH after adjustment** | **ml of 30% NaOH used** |
|---|---|---|---|
| 0 | 5.94 | 8.05 | 2.1 ml |
| 1 | 6.87 | 8.07 | 1.5 ml |
| 2 | 7.46 | 8.06 | 1.3 ml |
| 3 | 7.58 | 8.05 | 1.1 ml |
| 4 | 7.71 | 8.06 | 1 ml |
| 5 | 7.84 | 8.06 | 0.6 ml |
| 6 | 7.97 | 8.05 | 0.3 ml |
| 6.5 | 7.99 | - | - |

The starting pH measured each hour was adjusted with a 30% NAOH solution to a value of about 8.05 (pH after adjustment).

When the enzymatic reaction is complete, the suspension is centrifuged (4500 rpm for 10 min) and the supernatant is discarded.

The pellet thus obtained is analyzed and has the following characteristics: 15.68% dry substance, 36.29% protein on dry substance and 4.75% ash on dry substance.

The pellet is suspended by adding a quantity of water equal to the one discarded after the first centrifugation.

The suspension thus obtained is kept under stirring for 15 minutes and then centrifuged (4500 rpm for 10 min).

The supernatant is discarded and the pellet obtained mainly consists of a yeast cell wall composition.

The composition has the following characteristics: 12.05% dry substance, 24.57% protein on dry substance and 3.91% ash on dry substance.

The composition is suspended in water up to a 8% dry weight; in this specific case, 0.5 parts of osmotized water are added per each part of the composition having 12.05% of dry substance. The suspension is heated and maintained at 81°C for spray drying.

The pilot spray dryer has been set at an input temperature of 190°C and an outlet temperature of 91°C.

The obtained powder had the following characteristics: 13.63% moisture, 24.17% protein on dry substance and 3.79% ash on dry substance.

The polysaccharide content was analyzed, the composition being characterized by 21.8 total glucans, including 1.4 g of α-glucans and 20.4 g of β-glucan, and 6.5 of total mannans calculated on 100 g of final composition.

The process yield was 36.17%, i.e., 89.35 g of dry exhausted yeast yielded 32.317 g of dry cell walls.

## Claims

1. A process for the production of a composition consisting of yeast cell walls, which comprises steps of:
a) dispersing exhausted yeast in an aqueous medium to obtain a first suspension of yeast cells, said exhausted yeast consisting of yeast cells biomass recovered at the end of processes for the production of yeast extracts and/or ribonucleotides;
b) treating said first suspension having a dry weight of between 5% and 12% with a proteolytic enzyme at a temperature comprised between 50°C and 70°C, for 6 to 10 hours, in an aqueous medium containing a basic compound and having a pH between 7.5 and 9.0;
c) centrifuging said first suspension obtaining a first pellet and a supernatant;
d) dispersing said first pellet in an aqueous medium, obtaining a second suspension of yeast cells;
e) centrifuging said second suspension to obtain a second pellet having a protein content comprised between 20% and 30 % on dry substance, which constitutes said composition consisting of yeast cell walls.

2. The process according to claim 1, wherein said process comprises a further step f) of drying said second pellet, which constitutes said composition consisting of yeast cell walls obtained in step e).

3. The process according to claim 1 or 2, wherein said exhausted yeast is a Crabtree negative yeast, preferably belonging to the *Kluyveromyces* genus.

4. The process according to any one of claims 1-3, wherein said step b) of treating with a proteolytic enzyme is performed at a temperature comprised between 55°C and 65°C, preferably at 60°C.

5. The process according to any one of claims 1-4, wherein in said step b) of treating with a proteolytic enzyme, said suspension of exhausted yeast cells has a dry weight of between 7% and 12%.

6. The process according to any one of claims 1-5, wherein said step b) of treating with a proteolytic enzyme is performed at a pH of between 8.0 and 8.2.

7. The process according to claim 6, wherein said basic compound is a strong base, preferably an alkali or alkaline-earth hydroxide.

8. The process according to claim 7, wherein said alkali or alkaline-earth hydroxide is selected from sodium hydroxide, calcium hydroxide and potassium hydroxide, and is preferably sodium hydroxide.

9. The process according to claim 7 or 8, wherein said strong base is sodium hydroxide in aqueous solution with a concentration comprised between 20% and 40%, preferably between 25% and 35%, more preferably at a concentration of 30%.

10. A composition consisting of yeast cell walls having a content of glucans between 20% and 24%, a content of mannans between 5.5 and 7.5% and a protein content between 20% and 30% by weight, obtainable by the process according to any one of claims 1-9.

11. Use of the composition according to claim 10 as a food supplement.

12. Use of the composition according to claim 10 as a feed additive.

## Patentansprüche

1. Verfahren zur Herstellung einer Zusammensetzung, die aus Hefezellwänden besteht, umfassend die Schritte von:
a) Dispergieren von erschöpfter Hefe in einem wässrigen Medium, um eine erste Suspension von Hefezellen zu erhalten, wobei die erschöpfte Hefe aus Hefezellenbiomasse besteht, die am Ende von Verfahren zur Herstellung von Hefeextrakten und/oder Ribonukleotiden wiedergewonnen wurde;
b) Behandeln der ersten Suspension aufweisend ein Trockengewicht zwischen 5% und 12% mit einem proteolytischen Enzym bei einer Temperatur, die zwischen 50°C und 70°C liegt, für 6 bis 10 Stunden in einem wässrigen Medium, das eine basische Verbindung enthält und einen pH-Wert zwischen 7,5 und 9,0 aufweist;
c) Zentrifugieren der ersten Suspension unter Erhalt eines ersten Pellets und eines Überstandes;
d) Dispergieren des ersten Pellets in einem wässrigen Medium, Erhalten einer zweiten Suspension von Hefezellen;
e) Zentrifugieren der zweiten Suspension, um ein zweites Pellet mit einem Proteingehalt zwischen 20 und 30 % auf Trockensubstanz zu erhalten, das die Zusammensetzung aus Hefezellwänden bildet.

2. Verfahren nach Anspruch 1, wobei das Verfahren einen weiteren Schritt f) des Trocknens des zweiten Pellets umfasst, der die Zusammensetzung aus Hefezellwänden bildet, die in Schritt e) erhalten werden.

3. Verfahren nach Anspruch 1 oder 2, worin die erschöpfte Hefe eine Crabtree negative Hefe ist, die vorzugsweise zur Gattung Kluyveromyces gehört.

4. Verfahren nach einem der Ansprüche 1-3, worin der Schritt b) der Behandlung mit einem proteolytischen Enzym bei einer Temperatur zwischen 55°C und 65°C, vorzugsweise bei 60°C, durchgeführt wird.

5. Verfahren nach einem der Ansprüche 1-4, wobei in dem Schritt b) des Behandelns mit einem proteolytischen Enzym die Suspension von erschöpften Hefezellen ein Trockengewicht zwischen 7% und 12% aufweist.

6. Verfahren nach einem der Ansprüche 1-5, worin der Schritt b) des Behandelns mit einem proteolytischen Enzym bei einem pH-Wert zwischen 8,0 und 8,2 durchgeführt wird.

7. Verfahren nach Anspruch 6, worin die basische Verbindung eine starke Base ist, vorzugsweise ein Alkali- oder Erdalkalihydroxid.

8. Verfahren nach Anspruch 7, worin das Alkali- oder Erdalkalihydroxid ausgewählt ist aus Natriumhydroxid, Calciumhydroxid und Kaliumhydroxid, und vorzugsweise Natriumhydroxid ist.

9. Verfahren nach Anspruch 7 oder 8, worin die starke Base Natriumhydroxid in wässriger Lösung mit einer Konzentration zwischen 20% und 40%, vorzugsweise zwischen 25% und 35%, bevorzugter bei einer Konzentration von 30% ist.

10. Zusammensetzung, bestehend aus Hefezellwänden mit einem Gehalt an Glucanen zwischen 20% und 24%, einem Gehalt an Mannanen zwischen 5,5 und 7,5% und einem Proteingehalt zwischen 20% und 30 Gewichts-%, erhältlich durch das Verfahren nach einem der Ansprüche 1-9.

11. Verwendung der Zusammensetzung nach Anspruch 10 als Nahrungsergänzungsmittel.

12. Verwendung der Zusammensetzung nach Anspruch 10 als Futtermittelzusatzstoff.

## Revendications

1. Procédé de production d'une composition consistant en des parois de cellules de levure, qui comprend les étapes de :
a) dispersion de levure épuisée dans un milieu aqueux pour obtenir une première suspension de cellules de levure, ladite levure épuisée consistant en une biomasse de cellules de levure récupérée à la fin de processus de production d'extraits de levure et/ou de ribonucléotides ;
b) traitement de ladite première suspension ayant un poids sec entre 5 % et 12 % avec une enzyme protéolytique à une température comprise entre 50 °C et 70 °C pendant 6 à 10 heures, dans un milieu aqueux contenant un composé basique et ayant un pH entre 7,5 et 9,0 ;
c) centrifugation de ladite première suspension permettant d'obtenir un premier culot et un surnageant ;
d) dispersion dudit premier culot dans un milieu aqueux permettant d'obtenir une seconde suspension de cellules de levure ;
e) centrifugation de ladite seconde suspension pour obtenir un second culot ayant une teneur en protéines comprise entre 20 % et 30 % par rapport à la substance sèche, qui constitue ladite composition consistant en des parois de cellules de levure.

2. Procédé selon la revendication 1, dans lequel ledit procédé comprend une autre étape f) de séchage dudit second culot, qui constitue ladite composition consistant en des parois de cellules de levure obtenues à l'étape e).

3. Procédé selon la revendication 1 ou 2, dans lequel ladite levure épuisée est une levure Crabtree négative, de préférence appartenant au genre *Kluyveromyces.*

4. Procédé selon l'une quelconque des revendications 1 à 3, dans lequel ladite étape b) de traitement avec une enzyme protéolytique est réalisée à une température comprise entre 55 °C et 65 °C, de préférence à 60 °C.

5. Procédé selon l'une quelconque des revendications 1 à 4, dans lequel à ladite étape b) de traitement avec une enzyme protéolytique, ladite suspension de cellules de levure épuisées a un poids sec compris entre 7 % et 12 %.

6. Procédé selon l'une quelconque des revendications 1 à 5, dans lequel ladite étape b) de traitement avec une enzyme protéolytique est réalisée à un pH compris entre 8,0 et 8,2.

7. Procédé selon la revendication 6, dans lequel ledit composé basique est une base forte, de préférence un hydroxyde alcalin ou alcalino-terreux.

8. Procédé selon la revendication 7, dans lequel ledit hydroxyde alcalin ou alcalino-terreux est sélectionné parmi l'hydroxyde de sodium, l'hydroxyde de calcium et l'hydroxyde de potassium, et est de préférence l'hydroxyde de sodium.

9. Procédé selon la revendication 7 ou 8, dans lequel ladite base forte est l'hydroxyde de sodium en solution aqueuse avec une concentration comprise entre 20 % et 40 %, de préférence entre 25 % et 35 %, de manière davantage préférée en une concentration de 30 %.

10. Composition consistant en des parois de cellules de levure ayant une teneur en glucanes entre 20 % et 24 %, une teneur en mannanes entre 5,5 et 7,5 % et une teneur en protéines entre 20 % et 30 % en poids, pouvant être obtenue par le procédé selon l'une quelconque des revendications 1 à 9.

11. Utilisation de la composition selon la revendication 10 en tant que complément alimentaire.

12. Utilisation de la composition selon la revendication 10 en tant qu'additif alimentaire.
